Europäisches Patentamt

European Patent Office

Office européen des brevets

(19)

(11) Publication number: **0 147 192**
**A2**

(12) # EUROPEAN PATENT APPLICATION

(21) Application number: **84308964.0**

(51) Int. Cl.⁴: **A 61 B 17/22**

(22) Date of filing: **20.12.84**

(30) Priority: **03.01.84 US 567506**
**17.12.84 US 682393**

(43) Date of publication of application:
**03.07.85 Bulletin 85/27**

(84) Designated Contracting States:
**AT BE CH DE FR GB IT LI LU NL SE**

(71) Applicant: **Intravascular Surgical Instruments, Inc.**
**145 Oak Street**
**US-Downington, PA 19335(US)**

(72) Inventor: **Kensey, Kenneth R.**
**2801 Kings Drive, Apt. 110**
**Chicago, Illinois, 60616(US)**

(74) Representative: **Shaw, Laurence**
**George House George Road**
**Edgbaston Birmingham B15 1PG(GB)**

(54) **Recanalisation catheter with cutter head.**

(57) A catheter (10, 10a, 100) to clear atherosclerotic block-ages in a blood vessel (18) includes at its distal end a rotary cutting head (58, 58a, 162) which is driven by pressurised fluid fed up a passageway (26, 32a, 106) in the catheter and acting on a turbine part (76, 76a, 114) adjacent the cutting head. Passageways (32, 30a, 108) are provided to return the flow of fluid with cut away material. Any inflatable balloon (34, 200) may be present to seal the vessel.

FIG 1

INTRAVASCULAR SURGICAL                1·

INSTRUMENTS, INC.                            Agents Ref: 1121

## RECANALISATION CATHETER

## WITH CUTTER HEAD

The invention relates to a recanalisation catheter having a
cutter head and for use in surgically removing or displacing
atherosclerotic and like deposits.

It is known to correct coronary atherosclerotic narrowing or
occlusion by revascularisation of the myocardium. This
bypass surgery is expensive and the patient undergoes a
period of morbidity. An alternative procedure is
angioplasty and in this an inflatable balloon at the end of
a catheter is introduced into the vascular system and passed
along the coronary artery to the site of the occlusion. The
balloon is then inflated to compress the plaque and so open
the narrowing. Angioplasty is limited in scope and can
result in complications such as artery blow out or distal
emboli spasm.

It is one general object of this invention to provide an
improved catheter and method of use for opening restrictions
in a vessel, typically a blood vessel, in a living being.

According to one aspect of the invention there is provided a catheter having at a distal end means for opening a restriction within a blood or like vessel in a living body characterised in that the means comprises a cutting head to reduce the deposit and in that passageways are provided in the catheter to supply means from the proximal end of the catheter to actuate the cutting head.

The size and shape of the components of the device and the size of the catheter will vary according to the application and use. Preferably, the diameter of the catheter should be kept close to the internal diameter of the artery or other vessel in which it is to be inserted to assure co-axial movement of the cutting head and limit the amount of the lateral movement of the cutting blades so as to avoid direct contact of them with the arterial wall. Co-axial movement of the apparatus is also aided by the positive pressure applied through the catheter which centres the cutting head in the artery.

Most preferably, the cutting head is adapted to be rotated by the supply of pressurised fluid from the proximal end of the catheter and which is arranged to act on turbine means associated with cutting head. It is preferred that the cutting head is mounted for rotation about a shaft

INTRAVASCULAR SURGICAL    3

INSTRUMENTS, INC.                    Agents Ref: 1121

projecting distally forward from a stationary body which includes the passageways arranged to direct pressurised fluid on to the turbine means. Preferably the turbine means is present in the stationary body or comprises a separate element.

In one preferred embodiment there is a number of passageways arranged in compound angular relation to the major axis of the body in which they are present to impart a spinning motion to the pressurised fluid passed therethrough and directed on to the turbine means. Preferably, and in another feature, the body includes jet slots arranged to direct pressurised fluid on to the turbine blades to rotate the cutting head.

The cutting head preferably includes a plurality of cutting blades having a negative or zero degree rake angle.

In a preferred embodiment, the catheter includes passageways arranged to transport restrictive material removed from the inside wall of the vessel by the cutting head to the proximal end of the catheter, and inducer passages are present to induce reverse flow of fluid toward the proximal end of the catheter.

In another preferred feature the body and the turbine part are housed in a manifold and the cutting head is mounted on the turbine part, and fluid constraining means are present to retain pressurised fluid within the catheter. In this case, it is preferred that the cutter blade is dimensioned relative to the fluid restraining means to form a slight space between the vessel wall and the entrance to the interface between the cutter and the end of the shroud.

In yet another preferred feature, an annular flexible member is present to be inflated to engage the inside of the vessel. This member may be disposed either proximally or distally of the head. It is also preferred that a tube extends through the catheter and beyond the distal end thereof for the flow of profusion fluid into the artery distally of the restriction.

The design of the device is quite unique in that positive fluid pressure can be used to power the cutting head, and the driving fluid can be used to infuse drugs during the procedure. Also, the driving fluid can be oxygenated to profuse the distal myocardium thus eliminating time pressure on the surgeon and reducing the likelihood of any technical error. Also, for example, streptokinase can be infused if thrombosis should form in the artery. Nitrates can also be infused for vasodilation, and calcium blockers may be used to prevent arterial spasm. In contrast to the angioplasty procedures, pressure is not applied to the arterial walls, thus eliminating many of the complications associated with angioplasty such as blowout, emboli, intimal tearing, etc.

The application of positive and negative pressure can be easily accomplished and controlled through procedures known from the triple lumen catheter in use in other surgical applications.

In another embodiment a recanalization catheter is arranged to provide high power to a small cutting head over a substantial distance from a source of power. Thus the catheter may be introduced into the body at the site of a large diameter passageway, e.g. a femoral artery, and extended through the body to the site of a restriction in a small, remotely located artery, e.g. coronary artery. The catheter includes two, serially-connected, elongated sections, namely, a distal section and a proximal section. The distal section is a short, flexible member including a rotary cutting head driven by a rotating wire extending through the length of the section. The wire drive section is of small diameter for negotiating small passageways, such as coronary arteries. The proximal end of the distal section is connected to the distal end of proximal section by a turbine coupling. The turbine coupling includes a turbine head to which the wire drive is coupled. The proximal section is arranged to transmit high power over a substantial distance to the distal section of the catheter. Thus the proximal section of a larger diameter, long catheter section which includes a first passage extending therethrough for carrying a turbine driving fluid under pressure down the section to the turbine coupling to cause its rotation, and a second return passageway. The catheter also includes a passageway for guide wire.

In a further aspect, the invention provides a method of opening a restriction in a blood or like vessel in a living body characterised by inserting a catheter according to the invention into a blood vessel of the living body, locating the distal end of the cutting head at the site of the restriction and supplying power from the proximal end of the catheter to actuate the cutting head.

In order that the invention may be well understood it will now be described, by way of example only, with reference to the accompanying drawings, in which

Figure 1 is an exploded perspective view of the distal end of one embodiment of the invention;

Figure 2 is a sectional view of an artery containing the
catheter of Figure 1 located at the site of an
atherosclerotic deposit;

Figure 2A is a sectional view taken on the line 2A-2A of
Figure 2;

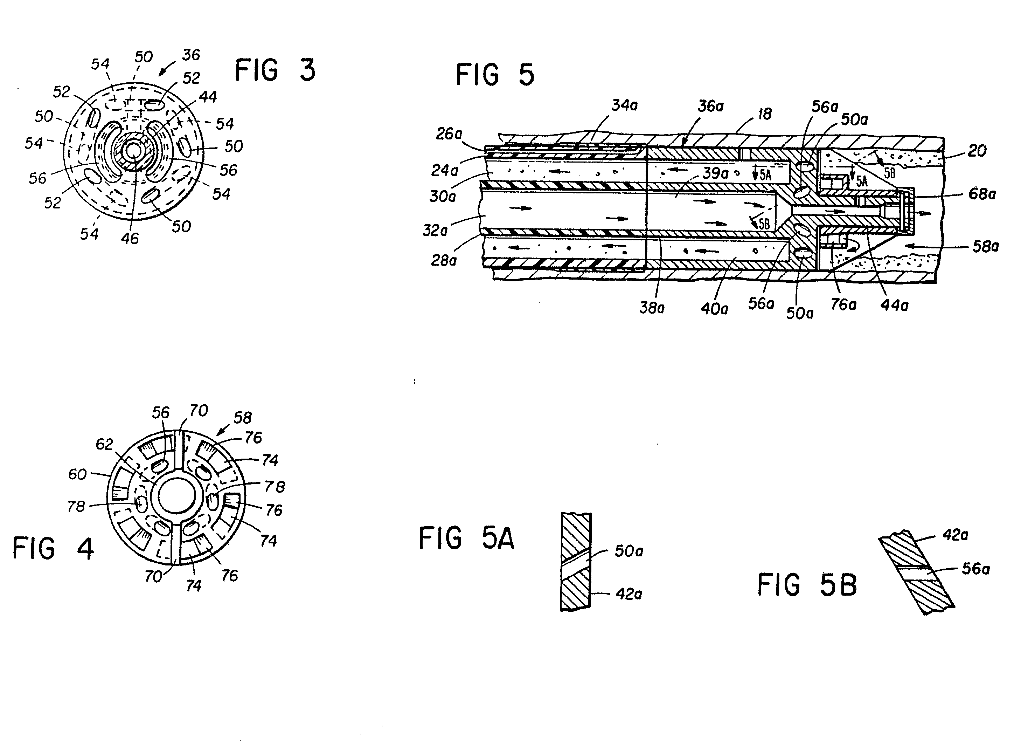

Figure 3 is an end view of the cutting end;

Figure 4 is an end view of the catheter with the cutting
head removed;

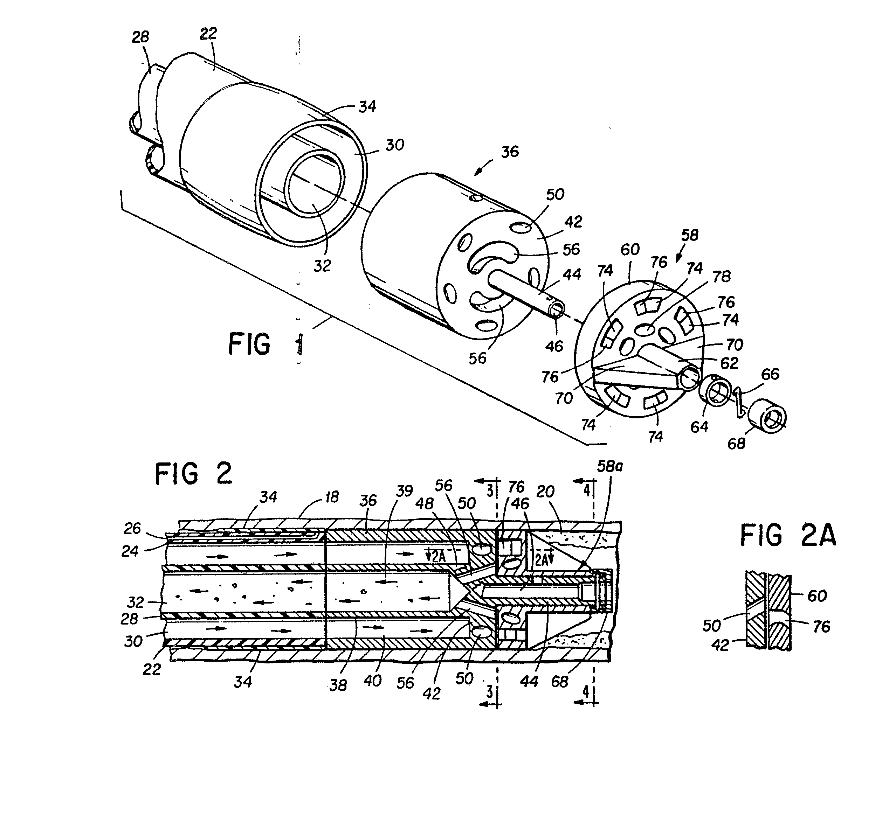

Figure 5 is a sectional view similar to Figure 2 containing
another embodiment of the invention;

Figures 5A and 5b are sectional views taken on the lines 5A-
5A and 5B-5B of Figure 5;

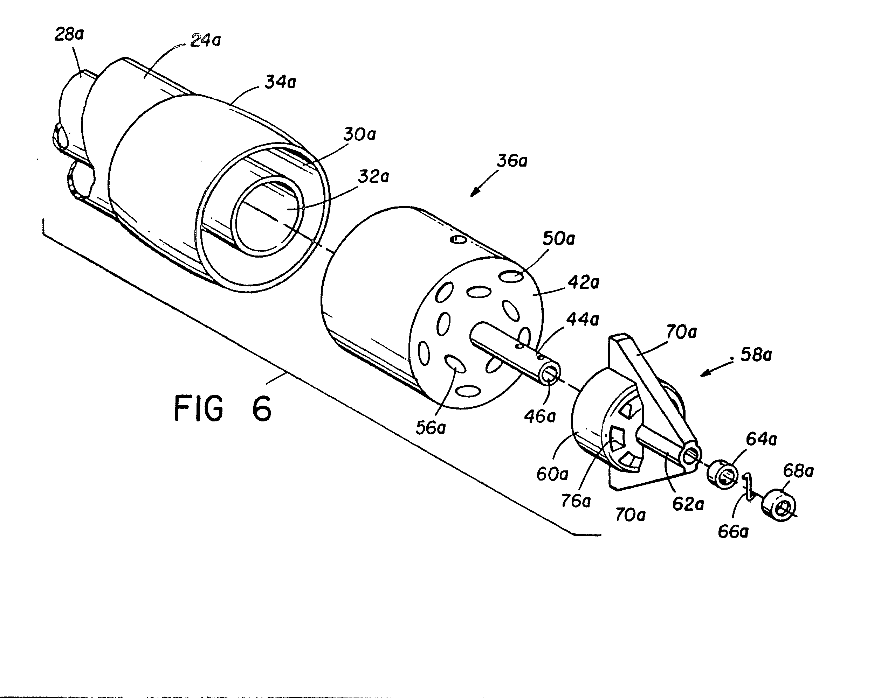

Figure 6 is an exploded perspective view of the device of
Figure 5;

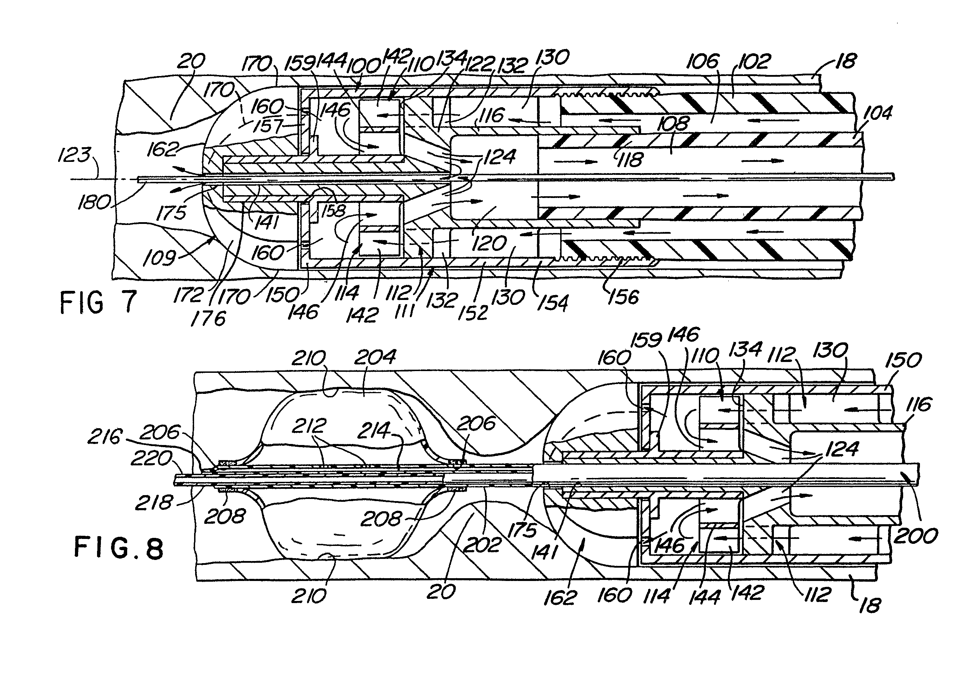

Figure 7 is a sectional view of an artery containing
another embodiment of the invention;

**0147192**

Figure 8 is a longitudinal sectional view, partially broken away, showing the device of Figure 7 and blocking means;

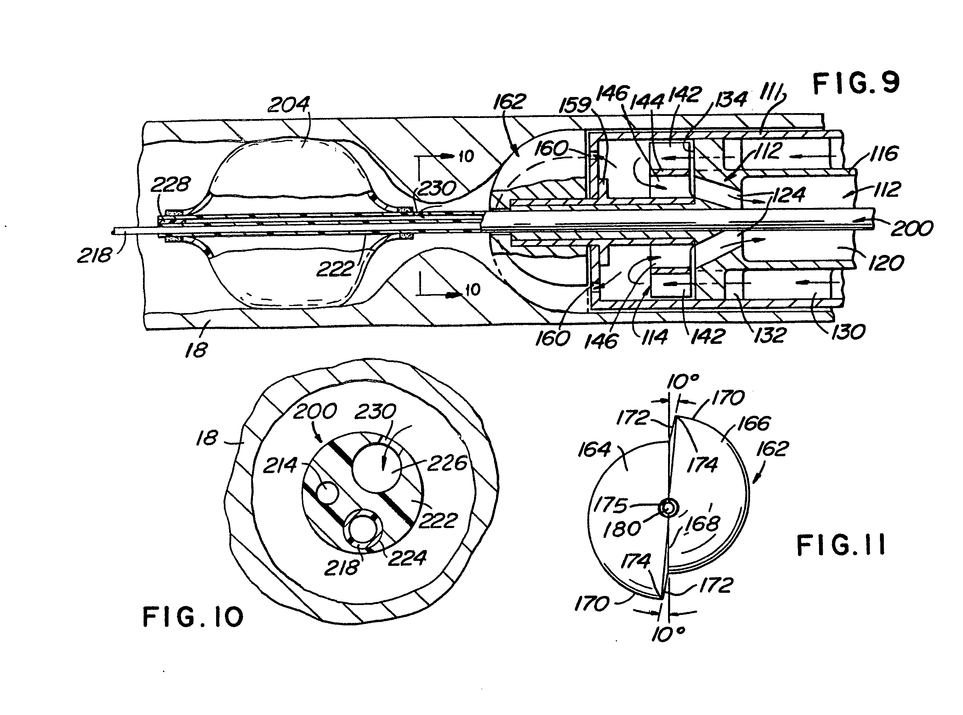

Figure 9 is a longitudinal sectional view similar to that of Figure 8;

Figure 10 is an enlarged sectional view taken along line 10-10 of Figure 9;

Figure 11 is an enlarged front view of the distal end of the device shown in Figure 7;

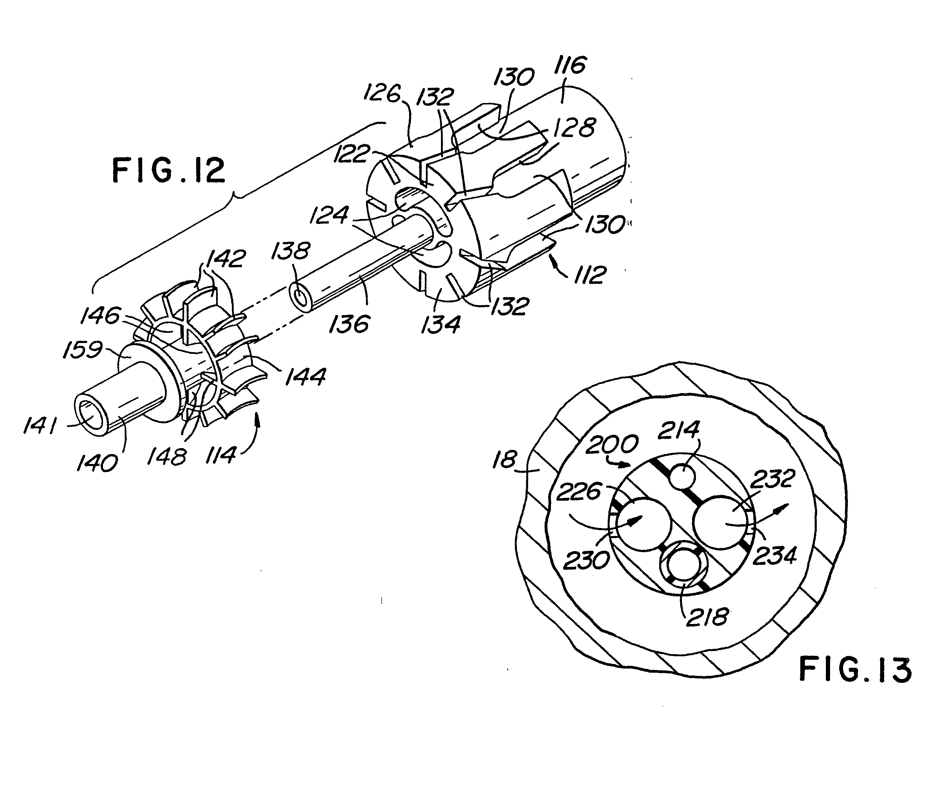

Figure 12 is an exploded perspective view of a portion of the working head of the catheter shown in Figures 7 to 9; and

Figure 13 is a sectional view similar to that of Figure 10 of a modified device.

The flexible recanalization catheter 10 of Figures 1 to 5 is to be introduced into the femoral artery at a point in the groin of the patient remote from the site of a blockage found in a coronary artery, for example. The device 10 is passed via the aorta into the heart and into the coronary artery to the site of the blockage.


In Figure 2, there is illustrated a coronary artery 18 which contains a partial occlusion or blockage caused by a deposit of atherosclerotic plaque 20. Figure 2 shows the distal end of the device 10 at the site of the blockage caused by the plaque 20. The device 10 consists of a multiple-walled, flexible catheter having an outer wall 22, and a first inner wall 24 which provides an outer passageway 26 extending the length of the catheter. The device 10 has an inner tube 28 spaced from the inner wall 24 to define a relatively large annular passageway 30 and a return passageway 32.


The outer passageway 26 terminates in a thin, flexible, annular member 34 the distal end of which is sealed. Pressurised fluid introduced into the outer passageway 26 at the proximal end of the device 10 will cause the flexible member 34 to expand or "balloon" about its entire circumference, as described below.


A rigid body 36 has an inner tube 38, the diameter of which corresponds to the diameter of tube 28, and is sealed fluid-tight at the distal end of the device. An annular passageway 40 is thus formed in head 36 and is in direct communication with the passageway 30. Inner tube 38 provides a passageway 39 that communicates with return
passageway 32 of the catheter. The body 36 also has a front
wall 42 from which extends a hollow shaft 44 which contains
a passageway 46 which extends through the front wall 42 and
communicates with annular passageway 40 via an angular
passageway 48. The front wall 42 of body 36 is relatively
thick, as shown in Figure 2, and has a plurality of
passageways 50. As best seen in Figure 3, each passageway
50 terminates at each end in an oval shaped opening, thus an
oval-shaped opening 52 at front of wall 42 and a
corresponding opening 54 in the inside face of wall 42. The
openings 52 and 54 are offset circumferentially, for reasons
explained below. The front wall 42 also contains on
opposite sides of the shaft 44 substantially kidney-shaped
passageways 56 which provide communication through front
wall 42 to the return passageway 39.

A rotary cutting head 58 is mounted for rotation on shaft
44. The head 58 has a disc portion 60 joined to a tubular
portion 62 which fits over the shaft 44. The cutting head
58 is retained on the shaft 44 by a thrust washer 64 and
wire 66 passing through holes in the outer end of shaft 44.
A bonnet 68 is press fitted over the end of the shaft 44 to
present a smooth uninterrupted leading surface.

The cutting head 58 has a pair of generally triangular cutting blades 70 extending radially outwardly in opposite directions from the tubular portion 62 and having cutting surfaces 72.

The disc 60 of the cutting head 58 also contains a plurality of outer passageways 74 in the wall of which are formed angular turbine blades 76. The outer passageways 74 correspond to the spacing of the openings 52 in the body 36 so that, because of the angularity of passageways 50, fluid flowing through passageways 50 will strike the angular turbine blades 76 and so impart rotating motion to the cutting head 58.

The cutting head 58 also contains a plurality of holes 78 which are angled relative to wall 42 to capture fluid in an axial pumping fashion and discharge the fluid into passageways 56 in head 36.

In use, the device is passed via the aorta into the coronary artery until it reaches the blockage. The device may be guided using a fluoroscope, and contrast medium can be introduced through the passageway 30. A suitable fluid is supplied under pressure through the passageway 30 of the catheter to rotate the cutting head 58 by application of the pressure to the angular turbine blades 76. Simultaneously, the angled holes 78 induce return flow to the proximal end of the device, acting like an axial flow pump. The pressure of the fluid is controlled, to ensure that the head is rotated at a steady rate. If necessary, negative pressure can be applied at the proximal end. The return flow will aspirate the particles of plaque being cut away by the rotating cutting head 58, and the pressure differential created by application of positive pressure through the passageway 30 will serve to pull the plaque 20 into a cutting position in the path of the cutting blades 72. During the cutting procedure, positive pressure introduced through the passageway 30 not only drives the rotating cutting head 58, but the fluid infused through this passageway can be oxygenated to eliminate distal inchemia during the procedure. The entire procedure is preferably performed under fluoroscopic control so that the surgeon can determine when the blockage has been completely cut away. When the blockage has been completely removed to the satisfaction of the surgeon, the device is withdrawn.

Although the invention provides minimal risk compared to angioplasty and bypass surgery, it is always possible that an artery wall weakened by disease or containing a congenital defect can break resulting in internal haemorrage.  If this occurs, the flexible member 34 can be inflated by fluid fed into passageway 26 to prevent blood loss until an appropriate surgical procedure can be conducted to correct the break in the arterial wall.

The body 36 and the cutting head 58 are the only rigid portions of the device and these do not interfere with easy passage of the instrument through a tortuous artery.  As a result a guiding catheter may not be needed and blind application of the device through a tortuous plaque infested artery may be adopted.

In the device of Figures 5 and 6, the innermost passageway of the catheter is used for the application of the positive pressurised fluid and the exterior annular passageway is used as the return passageway.  In this embodiment, parts corresponding to those of the first embodiment will be referred to by the same reference numeral of the first embodiment but suffixed "a".  The catheter has an outer wall 22a and a first inner wall 24a defining a passageway 26a, an inner tube 28a spaced from the inner wall 24a to define a large passageway 30a which is the return passageway.  Tube 28a also defines a central passageway 32a and the outer passageway 26a terminates in a thin, flexible annular member 34a the distal end of which is sealed.  A rigid body 36a has an inner tube 38a providing a passageway 40a in direct communication with the passageway 32a, body 36a has a front wall 42a which contains a plurality of circular passageways 50a that exit from the face of front wall 42a at an angle to the surface.  The passageways 50a communicate with the return passageway 40a.  Front wall 42a of body 36a also contain a plurality of inner passageways 56a.  These passageways 56a are in compound angular relationship to the axis of body 36a, having a radially outward direction and a vortex direction.  Fluid passing from passageway 39a exits from passageway 56a in a manner such as to impart a spinning action to the fluid and to the cutting head 58a.


The rotary cutting head 58a is mounted on the shaft 44a of body 36a, and has a main disc 60a and a tubular portion 62a.

A pair of cutting blades 70a diverge rearwardly from the tubular portion 62a.  The main disc 60a is of a smaller diameter than the diametral portion of the passageways 50a so that the passageways 50a communicate directly with the exterior of the device.  Because of the angularity of the passageways 56a, positive pressurised fluid  discharged from these passageways will impinge on the angled turbine blades 76a causing the cutting head 58a to rotate.  The  cutting head 58a is held in place on shaft 44a by a retaining ring and thrust washer 64a, a retaining wire 66a and a bonnet 68a.

In use, positive pressurised fluid is applied through the
inner passageway 32a of the catheter to rotate the  cutting
head 58a, while return is made through the outer annular
passageway 30a.

The coronary artery 18 of Figure 7 contains a partial
occulsion or restriction 20 formed by the deposit of
atherosclerotic plaque or some other material, such as waxy
and/or calcified atheroma, thickened and/or ulcerated
intima, etc.  The device 100 is located at the site of the
restriction 20.  The device 100 comprises a flexible
catheter that has an outer wall 102 and a first inner wall
104 which together define a relatively large annular outer
passage 106 extending substantially the entire length of the
catheter.  The passage 106 serves to carry a fluid to a
manifold assembly 110, to be described later.

The manifold assembly 110 is located within fluid
constraining means 111 (to be described in detail later) at
the distal end of the catheter.  The manifold assembly
(Figures 7 and 12) comprises a stationary body member 112
and a rotary turbine head 114.  The turbine head 114 serves
as the mount for the cutter 162.  The body member 112 is a generally cylindrical element which includes an elongated annular wall 116 extending longitudinally in the distal direction. The wall 116 is arranged for connection to the catheter inner wall 104 to receive fluid flowing therethrough. The free end 118 of the iner wall 104 is secured within the annular wall 116 as a snug fit so that the interior space 120 of the annular wall 116 is in fluid communication with the passageway 108. The distal end of the member 112 includes a front wall portion 122 from which the annular wall 116 projects. A pair of diametrically opposed kidney-shaped fluid return ports 124 are provided in the front wall. Each of the ports extends at an acute angle to the longitudinal central axis 123 of the catheter and communicates with the interior 120 of the member 112. Thus each return port is in fluid communication with the return passageway 108.

As shown clearly in Figure 12 the annular wall 116 includes a thickened peripheral wall portion 126 contiguous with the distal end of member 112 and in which are located peripherally disposed, fluid-supply slots 128. Each supply slot includes an elongated enlarged entrance 130 which terminates in a narrow jet slot 132. Each jet slot is disposed at an acute angle to the longitudinal axis of the device and terminates at the front face 134 of the member 112. An elongated cylindrical post bearing 136 projects distally from the centre of the front face of the member 112 and the kidney shaped fluid return ports 124 extend substantially about the post bearing. A central opening 138 extends through the bearing 136 and is in communication with the hollow interior 120 of the stationary member.

The turbine head 114 is a generally cylindrical member having an elongated cylindrical shaft portion 140 which serves as the mount for the cutter 162. The shaft 140 has a central bore 141 which receives the post bearing 136 of the stationary member 112. The proximal end of the turbine head 114 includes a plurality of turbine blades 142 extending outwards radially from a central hub portion 144. Each blade is slightly curved in the longitudinal direction. The central hub portion 144 of the turbine head includes angled curved passageways 146 formed between plural, equidistantly spaced, outwardly projecting, sharply angled arcuate walls 148 (see Figure 12). These passages serve as fluid return inducers. When the turbine head 114 is mounted on the post bearing 136 the inducer passages 146 of the turbine head are disposed opposite to the stationary kidney shaped return ports 124 while the turbine blades are disposed opposite the

INTRAVASCULAR SURGICAL          18

INSTRUMENTS, INC.                    Agents Ref: 1121

jet slots 132.

The manifold assembly 110 is secured on the distal end of
the catheter by the fluid constraining means 111 comprising
a shroud 150. The shroud constrains a substantial portion
of the turbine driving fluid within the catheter and takes
the form of a tubular sleeve having a cylindrical sidewall
152, the proximal end 154 of which is internally threaded
for securement to corresponding threads 156 on the distal
end of the catheter outer wall 102. The stationary member
112 of the manifold assembly is located within the shroud
150 so that the peripheral surface of the wall portions 126
between adjacent supply slots is in a tight, press-fitting
engagement with the interior surface of the shroud. As can
be seen in Figure 7 the front end of the shroud is in the
form of a planar endwall 157 having a central opening 158
through which the free end of the shaft 140 extends. A
disk-like thrust annulus 159 extends radially outward from
the shaft 140 at an intermediate point therealong and
engages the interior of the shroud front wall contiguous
with its central opening 158. The front wall of the shroud
also includes a plurality of small outlet ports 160 whose
function will also be described later.

Disposed on the free end of the shaft 140 is the rotary cutter 162.

The cutter 162 (Figures 7 and 11) comprises a solid element whose outer distal periphery is in the form of a pair of convex sections 164 and 166 which are slightly laterally offset from each other along a divider line 168. The intersection of the convex surface 170 of each section with a planar surface 172 contiguous with a divider line 168 forms an arcuate cutting edge or blade 172. The cutter may be made up of any number of sections, thereby forming a device having any number of arcuate blades, with each blade preferably including at least one portion having a negative or zero degree rake. In the embodiment shown herein each blade is at a negative rake angle of 10 degrees.

A central hole 175 extends through the cutter 162 and includes an enlarged bore 176 which extends into the body of the cutter and receives the shaft 140 of the turbine member 112.

The catheter 100 is guided into position within the artery 18 by insertion of a guidewire 180 in the femoral artery, which is then passed via the aorta into the coronary artery to the location of the partial occlusion or blockage 20. The catheter 100 is then threaded down the guidewire 180,

INTRAVASCULAR SURGICAL          20

INSTRUMENTS, INC.                          Agents Ref: 1121

via the opening 175 in the cutter, the opening 141 in the manifold assembly, and the hollow interior of inner wall 104, to a position wherein the cutter 162 is located immediately adjacent the proximal end of the restriction 20. Fluid is then introduced into the passage 106 by means (not shown) from a point adjacent the proximal end thereof. The fluid flows down the passage 106 in the direction of the arrows and is controlled by means (not shown) so that controlled positive pressure is applied to the manifold assembly. The fluid flows into the supply slots 128 between the outer surface of tube portion 116 and the inner surface of the shroud sidewall. The fluid then accelerates through the communicating jet slots 132 and exits those slots as plural jet streams, each extending at an acute angle to the longitudinal axis 123 of the catheter. These angularly directed jet streams impinge on the turbine blades 142 to impart rotary motion to the turbine head 114 and hence to the cutter 162.

In the absence of the shroud 150 the amount of fluid flow required to provide sufficient power for effectively driving the cutter 162 may be too much for some distally located tissues to absorb. The shroud 150 thus serves to constrain or contain most of the driving fluid within the catheter. A small portion of the driving fluid does exit from the distal end of the catheter through the shroud openings 160 and into the interface between the cutter 162 and the front wall of the shroud.  This action has the advantage of providing positive pressure to the wall of the artery, thereby causing the artery wall to move slightly outward radially, that is away from the device.  This reduces the risk of damage to the artery walls by the cutter and precludes fine fibrous tissue of the artery from gaining ingress into the interface where it could snag or spool up.

Moreover, the rotating cutter blades impart momentum to the

exiting fluid, which action applies further positive pressure to the artery walls, thereby further decreasing the risk of tissue snagging.


With the bulk of the fluid constrained within the catheter the angled oriention of the return inducer passages 146 act like an axial flow pump to induce a reverse flow of the fluid back toward the proximal end of the catheter.  In particular the fluid flows from the inducer passages 146 into the immediately adjacent kidney shaped return ports 124 and from there into the chamber 120 and into the return flow passage 108.


As can be seen by the arrows in Figure 7 some fluid also flows out of the distal end of the device via the aligned central openings 141 and 175.  This fluid can be used to profuse downstream (distal) tissue.  The fluid used to drive the catheter 100 may be oxygenated, may include drugs or medicines, or contrast media, or combinations thereof for introduction into the artery via the openings 160 and 175.

In use, the surgeon operates by advancing the catheter as its cutter rotates into the material making up the restriction so that the rotating cutter blades engage that material.  In some instances, e.g. hard or calcified deposits, the opening is created by the rotating cutting head actually cutting away or emulsifying particles of the material making up the restriction.  In other instances, e.g. waxy or soft deposits, the material of the restriction may merely be mechanically agitated, beaten or otherwise disturbed by the blades of the rotating cutter, whereupon an opening is created by the movement of the material without it actually being cut up or removed from the restriction. In either case there results an opening permitting the free flow of blood through the restriction.

As can be seen in Figure 7 the radial distance to the cutting edge 174 of each blade immediately adjacent to the proximal end of the cutter when measured from the logitudinal central axis 123 is slightly longer than the radial distance from that axis to the outside surface of the sidewall 154 of the shroud.  This feature ensures that a slight space is created between the inner surface of the artery wall and the entrance to the interface between the cutter and the end of the shroud, for detering any snagging or spooling action of the fibrous tissue of the artery wall within the interface.


The guidewire 180 may be replaced by a flexible hollow guide tube (not shown) for directly carrying fluids such as drugs etc. distally of the cutting head.


The catheter 100 of Figure 8 is used in combination with means 200 for blocking the artery 18 distally of the restriction to preclude any particles, e.g., emboli, of the restriction removed during the restriction opening process from flowing distally.  Thus, the embodiment of Figure 8 is of particular utility for surgical applications involving the removal of heavy atherosclerotic deposits in an artery that is sensitive to the escape of emboli downstream of the restriction.  One such application is the opening of restrictions in carotid arteries.


The blocking means 200 comprises a multi-wall tube 202 and a balloon 204.  The tube 202 extends the length of the catheter 100, through the catheter inner wall 104, the manifold assembly opening 141 and the cutter opening 175 and terminates in an end portion lying distally beyond the restriction 20. The balloon is a hollow, inflatable member having a pair of openings 206 through which a distal portion of the tube 202 extends. The wall portion of the balloon contiguous with the periphery of each opening 206 is sealed at 208 to the periphery of the outer surface of the tube 202. The balloon is formed of a material and is configured in size so that when it is inflated by a fluid, in a manner to be described later, it completely expands to fill the artery 18, that is the periphery 210 of the balloon engages the inner surface, e.g., intima, of the artery about the entire periphery of the artery and without stretching of the material forming the balloon. Inflation of the balloon is effected by means of inflation/deflation ports 212 provided in an inflation passageway 214 in the tube 202. The ports 212 are in communication with the interior of the balloon and with the passageway 214. The passageway 214 extends longitudinally down the tube 202 from a point (not shown) adjacent the proximal end of the catheter to a point 216 just distally of the balloon, at which point the passageway 214 is sealed. Fluid is supplied down the passageway 214 from a proximal location will inflate the balloon, which is deflated by drawing the fluid from the balloon interior out

INTRAVASCULAR SURGICAL          25

INSTRUMENTS, INC.                        Agents Ref: 1121

through passageway 214.

The tube 202 also includes a second passageway extending throughout the length of tube 202. A tube 218 also of the length of tube 202 is disposed within this second passageway and terminates at an open end 220 located distally of the balloon 204. The tube 218 serves as a passageway for the flow of a profusion fluid into the artery distally of the restriction as well as a guide (similar to guidewire 180) to facilitate the placement of the head just proximally of the restriction and with the balloon placed just distally thereof.

In use, the guide/profusion tube 218 is threaded through the artery to the site of the restriction, e.g., stenosis. The tube 202 with the balloon 204 leading is then threaded over the guide tube 218 until the balloon is just distally of the stenosis. Blood or oxygen saturated fluid is then passed through the tube 218 to flow out the open end 220 thereof so as to pass distally into the artery. The balloon 204 is inflated, by the passage of fluid through the inflation passageway 214, until the periphery of the balloon engages the interior of the artery. The catheter 100 is then passed over the tube 202 until its cutter 162 is located immediately adjacent the restriction. The turbine of the catheter is then driven by the introduction of fluid into
the passageway 106 while the cutter is advanced into the
restriction. Any particles or emboli of the stenosis cut
away or emulsified by the blades of the cutter are precluded
from flowing distally of the restriction by the expanded
balloon. Once the restriction has been opened the
recanalizing catheter 100 is removed, with balloon 204 still
expanded. Debris is then aspirated by flushing/aspirating
catheter (not shown) introduced into the artery over the
tube 202. Once flushing and aspiration is completed the
balloon 204 is deflated and the blocking means 200 is
removed.

The tube 202 may include another longitudinally extending
passageway to serve as a flushant return line (aspiration
line) to avoid the need for a separate flushing/aspirating
catheter. Thus, the blocking means 200 of Figures 10 and 11
comprises a balloon 204 mounted on a multi-wall tube 222
(Figure 10) which includes a longitudinally extending
passage 224 about one guide/profusion tube 218. The tube
222 also includes the passageway 214 and an aspiration or
suction return passageway 226. The passageway 226 is closed
at its end 228 but just proximally of the location of the
balloon 204 there is an access or entrance port 230 which is
the opening into which any emboli-bearing fluid passes down the tube 226.

In Figure 13 there is a sectional view of another multi-lumen tube for the blocking means 200 and which comprises a profusion/guide tube 218, the inflation/deflation passageway 214, and the aspiration passageway 226, and a flushant carrying passageway 232. The flushant passageway is similar in construction to passageway 226 and thus includes an outlet port 234 open at the site of the restriction. The outlet port is located adjacent the inlet port 230. A flushant fluid can be supplied down the passage 232 and out through port 234 to flush away any emboli or particles created at the site of the restriction.

The fluid supplied to drive the catheter turbine can also be used as the flushant as a portion of the fluid exits from the interior of the catheter at the location of the cutting head.

INTRAVASCULAR SURGICAL          1

INSTRUMENTS, INC.                              Agents Ref: 1121


## CLAIMS


1. A catheter having at a distal end means for opening a restriction within a blood or like vessel in a living body characterised in that the means comprises a cutting head (58, 58a, 162) to reduce the deposit (20) and in that passageways (26, 32a, 106) are provided in the catheter (10, 10a, 100) to supply means from the proximal end of the catheter to actuate the cutting head (58, 58a, 162).


2. A catheter according to Claim 1 characterised in that the cutting head (58, 58a, 162) is adapted to be rotated by the supply of pressurised fluid from the proximal end of the catheter and which is arranged to act on turbine means (76, 76a, 114) associated with cutting head (58, 58a, 106).


3. A catheter according to Claim 1 or 2, characterised in that the cutting head (58, 58a) is mounted for rotation about a shaft (46, 46a) projecting distally forward from a stationary body (36, 36a) which includes the passageways arranged to direct pressurised fluid on to the turbine means (76, 76a).

4. A catheter according to Claim 3 <u>characterised</u> <u>in</u> <u>that</u>
   the turbine means (76, 76a) is present in the stationary
   body (36, 36a).

5. A catheter according to Claim 3 <u>characterised</u> <u>in</u> <u>that</u>
   the turbine means (114, Figure 12), comprises a
   separate element.

6. A catheter according to any preceding Claim
   <u>characterised</u> <u>in</u> <u>that</u> there is a number of passageways
   (56a, Figures 5 and 6) arranged in compound angular
   relation to the major axis of the body (36a) in which
   they are present to impart a spinning motion to the
   pressurised fluid passed therethrough and directed on to
   the turbine means (76a).

7. A catheter according to any of Claims 1 to 5
   <u>characterised</u> <u>in</u> <u>that</u> the body (112, Figure 12)
   includes jet slots (132) arranged to direct
   pressurised  fluid on to the turbine blades (142)
   to rotate the  cutting head (162).

8. A catheter according to any preceding Claim
   <u>characterised</u> <u>in</u> <u>that</u> the cutting head (162) includes a plurality of cutting blades (172) having a negative or zero degree rake angle.

9. A catheter according to any preceding Claim characterised in that the catheter includes passageways (32, 30a, 108) arranged to transport restrictive material (20) removed from the inside wall of the vessel by the cutting head (58, 58a, 162) to the proximal end of the catheter (10, 10a, 100).

10. A catheter according to Claim 9 characterised in that inducer passages (146) are present to induce reverse flow, of fluid toward the proximal end of the catheter.

11. A catheter according to any preceding Claim characterised in that the body (112) and the turbine part (114) are housed in a manifold (110) and in that the cutting head (162) is mounted on the turbine part (114).

12. A catheter according to any preceding Claim characterised in that fluid constraining means (111) are present to retain pressurised fluid within the catheter.

13. A catheter according to Claim 12, characterised in that the cutter blade (174) is dimensioned relative to the fluid restraining means (111) to form a slight space between the vessel wall and the entrance to the interface between the cutter and the end of the shroud (150).

14. A catheter according to any preceding Claim characterised in that an annular flexible member (34, Figure 1 or 200, Figure 8) is present to be inflated to engage the inside of the vessel (18).

15. A catheter according to any preceding Claim characterised in that a tube (218, Figure 9) is present and extends through the catheter (100) and beyond the distal end thereof for the flow of profusion fluid into the artery (18) distally of the restriction (20).

16. A catheter according to any of Claims 1 and 7 to 10, 14 or 15 characterised in that the power means comprises a rotary wire extending the length of the catheter.

17. A method of opening a restriction in a blood or like vessel in a living body characterised by inserting a catheter according to any preceding Claim into a blood vessel of the living body, locating the distal end of the cutting head (58, 58a, 162) at the site of the restriction (20) and supplying power from the proximal end of the catheter to actuate the cutting head (58,

58a, 162).

FIG 1

FIG 2

FIG 2A

0147192

FIG 5

FIG 3

FIG 5A

FIG 5B

FIG 4

FIG 6

0147192

FIG 7

FIG.8

FIG.9

FIG.10

FIG.11

FIG.12

FIG.13